Europäisches Patentamt

European Patent Office

Office européen des brevets

(11) Numéro de publication: **0 292 410**
**A1**

## (12) DEMANDE DE BREVET EUROPEEN

(21) Numéro de dépôt: 88420159.1

(22) Date de dépôt: **17.05.88**

(51) Int. Cl.4: **A 61 N 5/06**

(30) Priorité: **18.05.87 FR 8707541**

(43) Date de publication de la demande:
**23.11.88 Bulletin 88/47**

(84) Etats contractants désignés:
**AT BE CH DE ES GB GR IT LI LU NL SE**

(71) Demandeur: **DIXWELL**
**17-19 rue Mazagran**
**F-69007 Lyon (FR)**

(72) Inventeur: **Aguettant, Jean**
**12 rue Nicolas de Lange**
**F-69005 Lyon (FR)**

(74) Mandataire: **Maureau, Philippe et al**
**Cabinet Germain & Maureau Le Britannia - Tour C 20, bld**
**Eugène Déruelle Boîte Postale 3011**
**F-69392 Lyon Cédex 03 (FR)**

(54) **Cabine de traitement par ultraviolets.**

(57) Cabine pour la pratique de l'irradiation par ultraviolets, dans un but thérapeutique ou de bronzage.

Ses parois verticales (2 à 5) sont constituées chacune par les tubes (12) associés par exemple à un cadre aéré (6) qui est équipé de moyens (10,11) de fixation et de connexion des tubes d'émission (12), cette cabine étant associée à un bloc électrique comportant par exemple les ballasts de ces tubes (12) et placé dans le socle (1) de la cabine ou à proximité de celle-ci.

FIG_1

EP 0 292 410 A1

Bundesdruckerei Berlin

## Description

## CABINE DE TRAITEMENT PAR ULTRAVIOLETS

La présente invention se rapporte à une cabine pour la pratique de l'irradiation par rayons ultraviolets, dans un but thérapeutique ou dans un but de bronzage.

Les cabines de bronzage ou plus généralement de photothérapie connues comportent quatre parois verticales pleines sur la face intérieure desquelles sont fixés les tubes d'irradiation, ainsi que leurs dispositifs électriques associés (starters et ballasts). Ces cabines ont pour inconvénients d'être lourdes, onéreuses, et d'évacuer difficilement la chaleur.

L'invention vise, en supprimant ces parois pleines et lourdes, à remédier à ces inconvénients. Elle se rapporte à une cabine de traitement par ultraviolets dont les parois verticales sont constituées chacune par les tubes d'irradiation eux-mêmes et par, au moins, un élément supérieur et un élément inférieur, destinés au support, à la fixation, et à la connexion électrique de ces tubes. Préférentiellement, un bloc électrique d'alimentation est associé à ces tubes, ce bloc électrique étant placé soit dans le socle de la cabine, soit à proximité de cette cabine.

L'invention sera bien comprise, et ses avantages et autres caractéristiques ressortiront, au cours de la description suivante d'un exemple non limitatif de réalisation, en référence aux dessins schématiques annexés dans lesquels:

Figure 1 est une vue en perspective de cette cabine, porte ouverte;

Figure 2 en est une vue en coupe horizontale selon II-II de figure 1, porte fermée;

Figure 3 est une vue en coupe selon III-III de figure 2;

Figure 4 est une vue en coupe selon IV-IV de figure 3, grille de protection retirée;

Figure 5 est une section tranversale d'un tube émissif, avec deux formes de réflecteur associé possibles;

Figures 6 à 9 sont des représentations schématiques, en vue de dessus, de quelques variantes de réalisation de cette cabine.

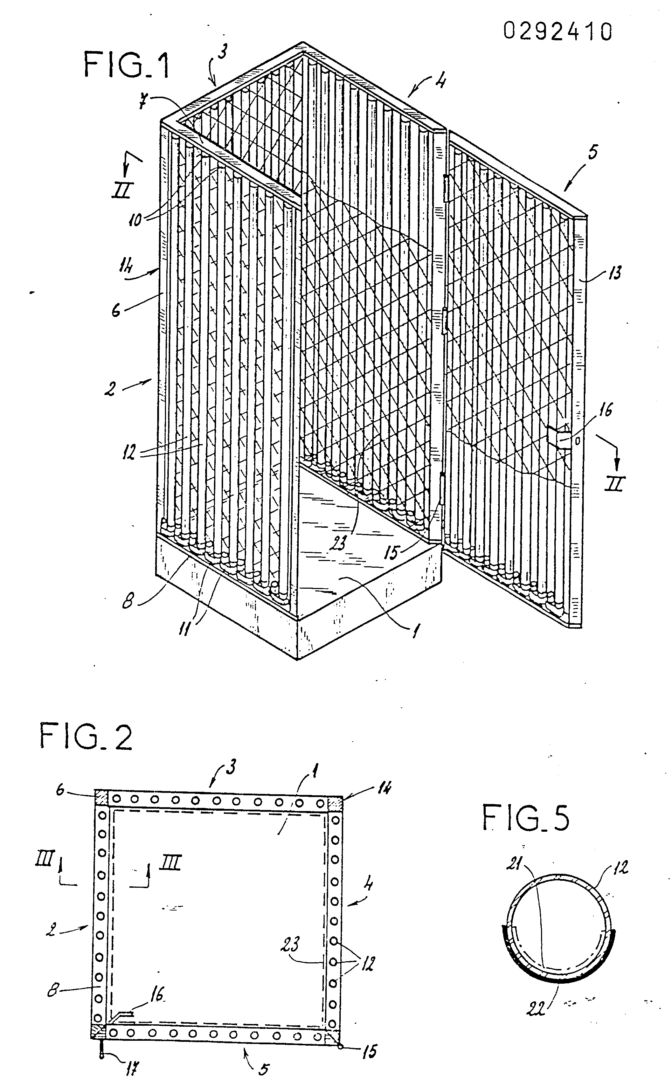

En se-reportant aux figures 1 à 4, cette cabine de bronzage est de section carrée et est de dimensions telles qu'un patient puisse se tenir debout sur son socle 1, tout en étant à une distance de ses parois verticales 2 à 5, qui est compatible avec le bronzage par tubes à ultraviolets. Les parois verticales 2 à 5, au lieu d'être pleines comme c'est le cas pour les cabines traditionnelles, sont chacune constituées par un cadre métallique rectangulaire, 6 par exemple pour la paroi 2, qui porte sur ses parties horizontales supérieure 7 et inférieure 8 des douilles 10,11 de fixation des tubes émissifs verticaux 12 équipant la paroi que ce cadre délimite.

Le cadre rectangulaire 13 qui délimite la paroi 5 est indépendant de l'armature métallique mécanosoudée 14 qui constitue les trois autres cadres délimitant les parois 2,3 et 4. Ce cadre 13 est relié à cette armature par trois charnières 15, de sorte que la paroi 5 constitue une porte d'entrée pour la cabine. cette porte étant munie d'une poignée intérieure 16 et d'une poignée extérieure 17.

Les douilles de fixation 10,11 sont choisies pour une fixation aisée sur les barres horizontales 7 et 8, cette fixation étant effectuée préférentiellement, comme c'est le cas dans cet exemple, sur les faces intérieures horizontales 18,19 des barres 7 et 8. La douille inférieure 11 est une douille avec porte-starter 20, par exemple une douille 341/F, apte à se fixer par deux vis perpendiculaires à sa base, et la douille supérieure est une douille de même type, mais sans porte-starter, par exemple une douille 340/F.

Sur les parties intérieures des quatre parois 2 à 5 sont fixées des grilles de protection mécanique 23, qui ont surtout une utilité en cas de chute accidentelle du patient.

Le socle 1 est creux et il contient tous les circuits d'alimentation électrique des tubes à ultraviolets 12, c'est-à-dire essentiellement les ballasts qui leur sont associés.

Comme représenté par des pointillés sur la figue 5, tous les tubes 12 ont la moitié de leur paroi interne revêtue d'une pellicule 21 de métal réfléchissant, tel que de l'Argent ou de l'Aluminium. Il est aussi possible d'utiliser des tubes 12 ordinaires, et de prévoir un réflecteur métallique indépendant 22 placé concentriquement et à faible distance de tube 12. La couche réflectrice 21, ou le réflecteur 22, ont pour rôle de réfléchir vers l'intérieur de la cabine les rayons émis par le tube 12.

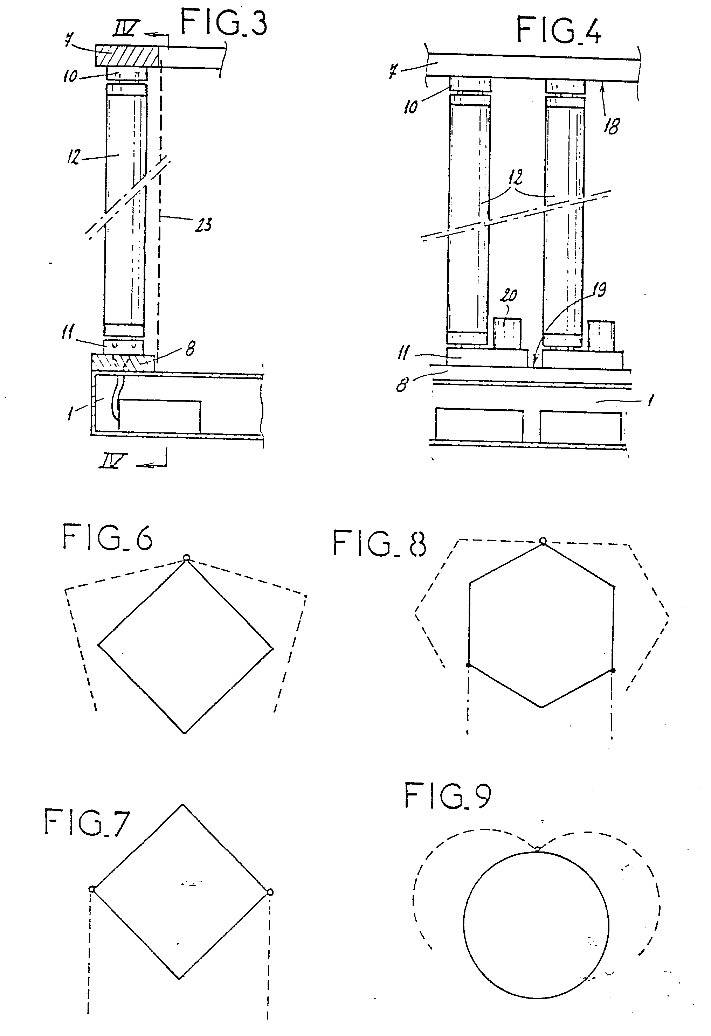

L'invention n'est pas limitée à l'exemple de réalisation qui vient d'être décrit. Les circuits électriques (ballasts) d'alimentation des tubes émissifs 12 pourraient être placés dans un bloc indépendant de la cabine, au lieu d'être placés dans le socle 1 (il n'est pas obligatoire d'avoir un socle). Ce bloc, avantageusement déplaçable sur roulettes, pourrait être prévu pour alimenter simultanément plusieurs cabines conformes à l'invention. Les formes et moyens d'ouverture de la cabine peuvent être variés: la forme restant carrée en section transversale, la cabine peut s'ouvrir en deux (figure 6) ou comporter deux portes (figure 7); la forme peut être polygonale et la cabine s'ouvrir en deux, ou autrement (figure 8); la forme peut âussi être ronde et la cabine peut s'ouvrir en deux par exemple (figure 9), etc...

## Revendications

1 - Cabine de traitement par rayons ultraviolets, caractérisée en ce que ses parois verticales (2 à 5) sont constituées chacune par les tubes d'émission (12) eux-mêmes et par, au moins, un élément supérieur (7) et un élément inférieur (8) destinés au support, à la fixation, et à la connexion électrique de ces tubes (12).

2 - Cabine de traitement par rayons ultraviolets selon la revendication 1, caractérisée en ce que ses parois verticales (2 à 5) sont constituées chacune par un cadre aéré (6) qui est équipé de moyens (10,11) de fixation et de connexion des tubes d'émission (12).

3 - Cabine selon l'une des revendications 1 ou 2, caractérisée en ce qu'elle est associée à un bloc électrique d'alimentation de ces tubes (12), placé dans le socle (1) de la cabine ou à proximité de celle-ci.

4 - Cabine selon la revendication 2, caractérisée en ce que les moyens de fixation et de connexion des tubes d'émission (12) sont constitués par des douilles (10,11) qui sont aptes à se fixer sur les faces intérieures (18,19) des parties horizontales supérieure (10) et inférieure (11) du cadre (6).

5 - Cabine selon la revendication 2 ou la revendication 4, caractérisée en ce que le cadre (6) est un cadre métallique rectangulaire.

6 - Cabine selon la revendication 5, caractérisée en ce qu'elle est constituée par un premier cadre indépendant (13) qui délimite une première paroi (5) apte à former la porte de la cabine, et par une armature métallique mécanosoudée (14) qui constitue les autres cadres délimitant les autres parois (2,3,4), des charnières (15) reliant ce cadre indépendant (13) à cette armature métallique (14).

7 - Cabine selon l'une des revendications 1 à 6, équipée d'un bloc électrique indépendant de la cabine, carctérisée en ce que ce bloc est apte à alimenter plusieurs cabines simultanément.

8 - Cabine selon l'une des revendications 1 à 7, caractérisée en ce que chaque tube est associé à un réflecteur (21 ou 22).

9 - Cabine selon l'une des revendications 1 à 8, caractérisée en ce que chaque paroi est équipée, sur sa partie intérieure, d'une grille de protection (23).

FIG.1

0292410

FIG.2

FIG.5

0292410

FIG.3

FIG.4

FIG.6

FIG.8

FIG.7

FIG.9

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | FR-A-2 353 684  (GTE SYLVANIA INC.) <br> * Page 4, ligne 13 - page 5, ligne 7; page 8, ligne 4 - page 9, ligne 35; figures * <br> --- | 1-6,8 | A 61 N   5/06 |
| A | FR-A-2 321 908  (WOLFF) <br> * Page 13, ligne 40 - page 14, ligne 5; figures 11,12 * <br> --- | 7 | |
| A | DE-A-3 013 081  (KRATZ) <br> * Page 7, lignes 3-5 * <br> ----- | 9 | |

|  | DOMAINES TECHNIQUES RECHERCHES (Int. Cl.4) |
|---|---|
| | A 61 N |

Le présent rapport a été établi pour toutes les revendications

| Lieu de la recherche | Date d'achèvement de la recherche | Examinateur |
|---|---|---|
| LA HAYE | 26-08-1988 | LEMERCIER D.L.L. |

EPO FORM 1503 03.82 (P0402)